# EUROPEAN PATENT APPLICATION

(11) **EP 4 458 987 A1**
(43) Date of publication of application: **06.11.2024**
(21) Application number: 21969698.6
(22) Date of filing: 31.12.2021
(51) Int. Cl.: C12Q 1/6869, C12N 9/12, G01N 27/447

(54) **HELICASE BCH1X AND USE THEREOF**

(71) Applicant: BGI Shenzhen, Shenzhen, Guangdong 518083 (CN)
(72) Inventor: XU, Xun, Shenzhen, Guangdong 518083 (CN); GUO, Fei, Shenzhen, Guangdong 518083 (CN); HU, Qiaoxia, Shenzhen, Guangdong 518083 (CN); LI, Denghui, Shenzhen, Guangdong 518083 (CN); CHEN, Junyi, Shenzhen, Guangdong 518083 (CN); WANG, Lele, Shenzhen, Guangdong 518083 (CN); ZHAO, Ziyu, Shenzhen, Guangdong 518083 (CN); JI, Zhouxiang, Shenzhen, Guangdong 518083 (CN); ZENG, Tao, Shenzhen, Guangdong 518083 (CN); WANG, Ou, Shenzhen, Guangdong 518083 (CN); LI, Yuxiang, Shenzhen, Guangdong 518083 (CN); DONG, Yuliang, Shenzhen, Guangdong 518083 (CN); ZHANG, Wenwei, Shenzhen, Guangdong 518083 (CN)
(74) Representative: Weickmann & Weickmann PartmbB
(86) International application number: PCT/CN2021/143662
(87) International publication number: WO 2023/123347

(57) **Abstract**

Provided is helicase BCH1X, which comprises an amino acid sequence represented by SEQ ID NO: 1 or 2. Further provided are a complex structure comprising helicase BCH1X and a binding moiety used for binding to a polynucleotide, and a use thereof in the control and characterization of a polynucleotide and in single molecule nanopore sequencing.

## Description

### Technical Field

The present application relates to the biological field, and specifically to a helicase and its use. More specifically, the present application relates to the helicase BCH1X and its use in the control and characterization of nucleic acids and in the nanopore sequencing.

### Background Art

Nanopore sequencing is a third-generation sequencing technology that has emerged in recent years. Due to its advantages such as long read length, high throughput, low cost, and portability, nanopore sequencing has brought disruptive changes to the gene sequencing industry. Nanopore sequencing technology is widely used in basic theoretical research in life sciences and biomedical clinical practice.

Nanopore sequencing is a sequencing technology based on electrical signals. It uses single-stranded nucleic acid molecules as sequencing units, and a nanopore (protein or solid-state) inserted in a membrane separates two electrolysis chambers filled with electrolyte. When voltage is applied to the two electrolytic chambers, a stable perforation current is generated. Different molecules entering the nanopore may block the flow of ions, which is called nanopore signal. When nucleic acids pass through the nanopore, the magnitude of the blocked current will vary due to different bases. By detecting the current fluctuation signal of the nanopore and analyzing the current signal through machine learning, the sequence of the nucleic acid that passes the nanopore can be determined. Nanopore sequencing technology has the following advantages: it can easily build a library without amplification; it has fast reading speed, and the reading speed for single-stranded molecules can reach tens of thousands of bases per hour; it has longer reading length and can usually reach several thousand bases; and it allows direct sequencing of methylated DNA or RNA.

Due to the extremely fast speed of nucleic acid molecules passing through nanopore channels, polynucleotide sequence information cannot be accurately obtained. Therefore, effectively reducing and controlling the movement of nucleic acid molecules through nanopores is a key technical issue in nanopore sequencing. Currently, the movement of nucleic acid molecules through nanopores can be controlled through polymerase amplification or helicase unwinding, and detection accuracy can be improved by increasing the residence time of nucleic acid molecules in the nanopores. Meanwhile, in nanopore sequencing, helicases need to have good salt tolerance and stability to maintain good sequencing speed and sequencing uniformity.

However, the helicases used in the currently commercialized nanopore sequencers are generally DDA helicases derived from bacteriophage T4, which have poor yield, stability, and salt tolerance. There is still a need in the art for novel helicases.

### Contents of the present invention

One object of the present disclosure is to provide a new helicase which can be used for characterization of nucleic acids, thereby solving the problems of poor salt tolerance and stability of conventional helicases, improving the yield of recombinantly expressed helicases, and significantly improving the accuracy of polynucleotide characterization.

In a first aspect, the present application provides a new helicase BCH1X, which is screened from a deep-sea metagenomic library (derived from the Shenzhen National Gene Bank). Compared with the helicases in the prior art, the helicase BCH1X has extremely high stability and salt tolerance. Moreover, the helicase BCH1X has a very high expression level in the recombinant protein expression system using *Escherichia coli,* and the yield is extremely high. In addition, the helicase BCH1X has a special pin structure, which enables it to have good single-stranded DNA binding and double-stranded DNA unwinding activities. The helicase can be used for control and characterization of nucleic acids and used for single-molecule nanopore sequencing.

In one embodiment, the helicase BCH1X comprises:
(i) the amino acid sequence as set forth in SEQ ID NO: 1 or 2; or
(ii) an amino acid sequence that has a sequence identity of at least 80%, at least 85%, at least 90%, at least 91%, at least 92%, at least 93%, at least 94%, at least 95%, at least 96%, at least 97%, at least 98%, or at least 99% as compared to the amino acid sequence as set forth in SEQ ID NO: 1 or 2 and has a helicase activity; or
(iii) an amino acid sequence that has no more than 20, 15, 10, 5, 4, 3, 2 or 1 amino acid difference as compared to the amino acid sequence as set forth in SEQ ID NO: 1 or 2 and has a helicase activity.

In one embodiment, the amino acid difference comprises an amino acid substitution, deletion and/or insertion or N-terminal and/or C-terminal extension, provided that the amino acid sequence having the amino acid difference retains the helicase activity of SEQ ID NO: 1 or 2. Preferably, the amino acid substitution is a conservative amino acid substitution.

In a preferred embodiment, the helicase BCH1X comprises the following amino acid fragment:
GTIHX FLNLKLDXGF Y1DDGY2ADNVY2 TKXKLVY3NKY4 NECL
wherein: X represents any amino acid residue; Y1=A or G; Y2=T or S; Y3=V or L; Y4=F or Y.

The amino acid fragment represents a key region for the helicase BCH1X to show better performance in sequencing. Specifically, the amino acid fragment is an important segment related to unwinding speed in the protein structure.

In one embodiment, the helicase BCH1X consists of the amino acid sequence as set forth in SEQ ID NO: 1 or 2.

In a specific embodiment, the helicase BCH1X as set forth in SEQ ID NO: 1 is named BCH105, and the helicase BCH1X as set forth in SEQ ID NO: 2 is named BCH178. Both BCH105 (SEQ ID NO: 1) and BCH17 (SEQ ID NO: 2) contain the amino acid fragment.

The helicase or complex structure thereof of the present disclosure can move a target polynucleotide through a nanopore in a controllable and stepwise manner through the magnetic field generated by an external voltage, thereby controlling the speed at which the polynucleotide passes through the nanopore, and obtaining a recognizable current level. In addition, the helicase BCH1X or complex structure thereof is able to function effectively under a high salt concentration and have an extremely high stability.

The helicase or complex structure thereof of the present disclosure has a very high expression level when recombinantly expressed (for example, expressed in *Escherichia coli*), and can be easily obtained at a high yield.

In a second aspect, the present application provides a nucleotide sequence encoding the helicase BCH1X of the first aspect.

In one embodiment, the nucleotide sequence encoding the helicase BCH1X comprises a nucleotide sequence encoding the following amino acid sequence:
(i) the amino acid sequence as set forth in SEQ ID NO: 1 or 2; or
(ii) an amino acid sequence that has a sequence identity of at least 80%, at least 85%, at least 90%, at least 91%, at least 92%, at least 93%, at least 94%, at least 95%, at least 96%, at least 97%, at least 98%, or at least 99% as compared to the amino acid sequence as set forth in SEQ ID NO: 1 or 2 and has a helicase activity; or
(iii) an amino acid sequence that has no more than 20, 15, 10, 5, 4, 3, 2 or 1 amino acid difference as compared to the amino acid sequence as set forth in SEQ ID NO: 1 or 2 and has a helicase activity.

In a preferred embodiment, the nucleotide sequence encoding helicase BCH1X comprises a nucleotide sequence encoding the following amino acid fragment:
GTIHX FLNLKLDXGF Y1DDGY2ADNVY2 TKXKLVY3NKY4 NECL
wherein: X represents any amino acid residue; Y1=A or G; Y2=T or S; Y3=V or L; Y4=F or Y.

In one embodiment, the nucleotide sequence can be codon-optimized for a cell used for expression to obtain a desired expression level in the cell.

In a third aspect, the present application provides a recombinant vector comprising the nucleotide sequence of the second aspect of the present application.

In one embodiment, the recombinant vector is a recombinant expression vector, for example, a recombinant expression vector suitable for expression in a prokaryotic or eukaryotic cell.

The prokaryotic cell includes, but is not limited to: *Escherichia coli* cell, *Bacillus subtilis* cell, etc.

The eukaryotic cell includes, but is not limited to: yeast cell, insect cell, mammalian cell (e.g., CHO cell, HEK293 cell), etc.

Preferably, the recombinant expression vector is suitable for expressing the helicase BCH1X in an *Escherichia coli* cell.

Preferably, in order to make purification of the helicase more convenient, a purification tag can be added to the helicase. When the tag needs to be removed, the tag can be removed by a chemical method or enzymatic reaction.

In a fourth aspect, the present application provides a cell comprising the nucleotide sequence of the second aspect or the recombinant vector of the third aspect. The cell may be a prokaryotic cell or eukaryotic cell, such as *Escherichia coli* cell, *Bacillus subtilis* cell, yeast cell, insect cell, mammalian cell (e.g., CHO cell, HEK293 cell), etc.

In a fifth aspect, the present application provides a complex structure, the complex structure comprises the helicase BCH1X of the first aspect and a binding moiety for binding a polynucleotide, wherein the helicase BCH1X is attached to the binding moiety, and the complex structure is capable of controlling the sequence of the polynucleotide.

Preferably, the complex structure has a natural structure or a non-natural structure.

In a specific embodiment of the present application, the complex structure has an artificially manufactured non-natural structure.

Preferably, the binding moiety may be a binding moiety capable of binding to a base of a polynucleotide, and/or a binding moiety capable of binding to a sugar of a polynucleotide, and/or a binding moiety capable of binding to a phosphate of a polynucleotide. Those skilled in the art can select an appropriate binding moiety according to a specific need.

The complex structure of the present application is an effective tool for controlling movement of a polynucleotide during sequencing. The helicase-containing complex structure of the present application is capable of stably binding to a polynucleotide and will not be separated from the polynucleotide during the sequencing process. The complex structure can provide a greater read length of the polynucleotide when controlling translocation of the polynucleotide through the nanopore. In a buffer, the binding of the binding moiety to the polynucleotide is compatible with the strand sequencing and the characterization process of the polynucleotide. Compared with a standard physiological level, the binding moiety has a better activity at a high salt concentration (e.g., 0.3 to 1 M KCl) because of its good salt tolerance, and improvement of the binding moiety of the complex structure can improve the synthesis ability, stability and half-life.

Preferably, the helicase and the binding moiety are bound or attached via their terminal amino acids. For example, the amino end of the binding moiety is bound or attached to the carboxy end of the helicase, or the carboxy end of the binding moiety is bound or attached to the amino end of the helicase. Further preferably, the binding moiety is inserted into the sequence of the helicase. Such a structure can well combine the helicase and the binding moiety through two points.

To make purification of the complex structure easier, a tag may be added to the complex structure. When the tag needs to be removed, it can be removed by a chemical method or enzymatic reaction.

In a sixth aspect, the present application provides a use of helicase BCH1X in controlling and characterizing a nucleic acid, or in a single-molecule nanopore sequencing. The helicase BCH1X or complex structure thereof of the present application can control the movement speed of a target polynucleotide through a nanopore.

In a seventh aspect, the present application provides a method for controlling and characterizing a target polynucleotide, the method comprising the following steps:
(a) contacting the target polynucleotide with a pore, and a helicase or complex structure thereof, such that the helicase or complex structure thereof controls the movement of the target polynucleotide through the pore; and
(b) obtaining one or more characteristics of a nucleotide in the target polynucleotide when it interacts with the pore, thereby characterizing the target polynucleotide;

wherein the helicase is the helicase BCH1X according to the first aspect of the present application, and the complex structure comprises the helicase BCH1X and a binding moiety for binding to the polynucleotide,
the one or more characteristics may be selected from, but are not limited to: a change in current signal magnitude, a change in current signal duration, a change in voltage signal magnitude, a change in voltage signal duration, etc.

In one embodiment, the method for controlling and characterizing a target polynucleotide is a method for a single-molecule nanopore sequencing.

In an eighth aspect, the present application provides a kit for controlling and characterizing a target polynucleotide, and the kit comprises the helicase BCH1X or a complex structure thereof and a pore. Preferably, the pore is a nanopore.

Preferably, the kit comprises multiple helicases or multiple complex structures, and multiple pores.

Preferably, the pore is a transmembrane pore, and the transmembrane pore is a biological pore, a solid-state pore, or a biological-solid-state hybrid pore. Further preferably, the biological pore is selected from the group consisting of α-hemolysin protein (α-HL), *Mycobacterium smegmatis* porin A (MspA), curli-specific transport channel protein (CsgG), type III secretion system protein (InvG), etc.

Preferably, the kit further comprises a chip comprising a lipid bilayer. The pore spans the lipid bilayer.

Preferably, the kit of the present application comprises one or more lipid bilayers, and each lipid bilayer comprises one or more of the pores.

Preferably, the kit of the present application also comprises a reagent or device for characterizing the target polynucleotide. Preferably, the reagent comprises a buffering agent, or an enzyme or buffer required for PCR amplification.

In one embodiment, the kit is a kit for a single-molecule nanopore sequencing.

In a ninth aspect, the present application also provides a sensor for characterizing a target polynucleotide, comprising a complex formed by a pore and the helicase BCH1X or complex structure thereof, wherein the pore is able to interact with a target polynucleotide, thereby forming a sensor for characterizing the target polynucleotide.

Preferably, the pore and the helicase BCH1X or complex structure thereof are contacted in the presence of the target polynucleotide, and an electric potential is applied across the pore. The electric potential may be selected from voltage potential or electrochemical potential.

Preferably, the pore is covalently linked to the helicase or the complex structure.

In a tenth aspect, the present application provides a device for characterizing a target polynucleotide, the device comprising the helicase BCH1X or complex structure thereof, and a pore.

Preferably, the device comprises a sensor device that supports the pore and can transmit a signal of the interaction between the pore and a polynucleotide, and at least one memory for storing the target polynucleotide, and a solution required for performing the characterization.

Preferably, the device comprises multiple helicases or multiple complex structures, and multiple pores.

Preferably, the pore is a transmembrane pore, and the transmembrane pore is a biological pore, a solid-state pore, or a biological-solid-state hybrid pore. Further preferably, the biological pore is selected from the group consisting of α-hemolysin protein (α-HL), *Mycobacterium smegmatis* porin A (MspA), curli-specific transport channel protein (CsgG), type III secretion system protein (InvG), etc.

In an eleventh aspect, the present application provides a method for preparing the helicase BCH1X described in the first aspect or a complex structure thereof, the method comprising: constructing a recombinant expression vector for expressing the helicase BCH1X or complex structure thereof, transforming the recombinant expression vector into an appropriate host cell for recombinant expression.

In one embodiment, the host cell may be a prokaryotic or eukaryotic cell, such as *Escherichia coli* cell, *Bacillus subtilis* cell, yeast cell, insect cell, mammalian cell (e.g., CHO cell, HEK293 cell), etc.

Those skilled in the art can select an appropriate expression vector and host cell according to an actual need.

"Nucleotide" in the present application includes, but is not limited to: adenosine monophosphate (AMP), guanosine monophosphate (GMP), thymidine monophosphate (TMP), uridine monophosphate (UMP), cytosine monophosphate (CMP), cyclic adenosine monophosphate (cAMP), cyclic guanosine monophosphate (cGMP), deoxyadenosine monophosphate (dAMP), deoxyguanosine monophosphate (dGMP), deoxythymidine monophosphate (dTMP), deoxyuridine monophosphate (dUMP) and deoxycytidine monophosphate (dCMP). Preferably, the nucleotide is selected from the group consisting of AMP, TMP, GMP, CMP, UMP, dAMP, dTMP, dGMP, or dCMP.

The "conservative amino acid substitution" used in the present application refers to a substitution of an amino acid by another amino acid within the same category, for example, a substitution of an acidic amino acid by another acidic amino acid, a substitution of a basic amino acid by another basic amino acid, or a substitution of a neutral amino acid by another neutral amino acid.

For example, amino acids can be categorized according to the properties of the side chains thereof:
(1) hydrophobic side chain: norleucine, Met, Ala, Val, Leu, Ile;
(2) neutral hydrophilic side chain: Cys, Ser, Thr, Asn, Gln;
(3) acidic side chain: Asp, Glu;
(4) basic side chain: His, Lys, Arg;
(5) side chain affecting chain orientation: Gly, Pro;
(6) aromatic side chain: Trp, Tyr, Phe.

Conservative amino acid substitution may refer to that one amino acid in the above group is replaced by another amino acid in the same group. The conservative amino acid substitution will basically not change the activity of the amino acid sequence of the present application.

Exemplary conservative amino acid substitutions are shown in Table A below:

**Table A: Exemplary substitutions and conservative substitutions**

| Original residue | Exemplary substitution | Conservative substitution |
|---|---|---|
| Ala (A) | Val; Leu; Ile | Val |
| Arg (R) | Lys; Gln; Asn | Lys |
| Asn (N) | Gln; His; Asp, Lys; Arg | Gln |
| Asp (D) | Glu; Asn | Glu |
| Cys (C) | Ser; Ala | Ser |
| Gln (Q) | Asn; Glu | Asn |
| Glu (E) | Asp; Gln | Asp |
| Gly (G) | Ala | Ala |
| His (H) | Asn; Gln; Lys; Arg | Arg |
| Ile (I) | Leu; Val; Met; Ala; Phe; norleucine | Leu |
| Leu (L) | norleucine; Ile; Val; Met; Ala; Phe | Ile |
| Lys (K) | Arg; Gln; Asn | Arg |
| Met (M) | Leu; Phe; Ile | Leu |
| Phe (F) | Trp; Leu; Val; Ile; Ala; Tyr | Tyr |
| Pro (P) | Ala | Ala |
| Ser (S) | Thr | Thr |
| Thr (T) | Val; Ser | Ser |
| Trp (W) | Tyr; Phe | Tyr |
| Tyr (Y) | Trp; Phe; Thr; Ser | Phe |
| Val (V) | Ile; Leu; Met; Phe; Ala; norleucine | Leu |

### Brief Description of the Drawings

The embodiments and advantages of the present disclosure will be more apparent in conjunction with the following drawings.
Fig. 1 shows the results of purification of the helicase BCH105 by the molecular sieve Superdex 200. Lane M represents the molecular weight marker, lanes 1 to 9 represent molecular sieve purification fractions 1 to 9, and fractions 6 to 9 are selected and combined as the protein sample to be tested for subsequent experiments.
Fig. 2 shows the binding results of BCH105 to ssDNA.

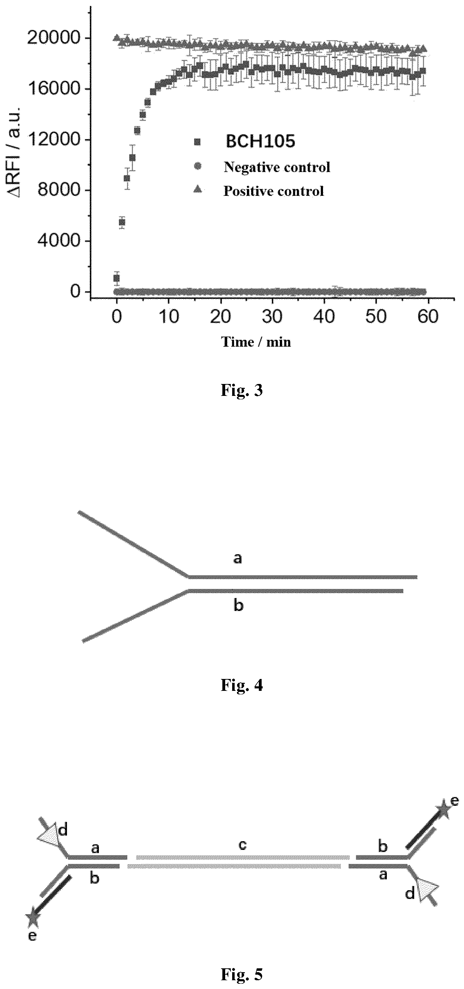

Fig. 3 shows the verification results of the unwinding activity of BCH105. Fluorescence quenching method was used to detect the double-stranded DNA unwinding activity of BCH105 protein. A double-stranded DNA with 5'-overhang formed by annealing a single-stranded DNA (labeled with a BHQ-1 quenching group at the 3' end) to another single-stranded DNA (labeled with a FAM fluorophore at the 5' end) was used as a substrate, and BCH105 protein was obtained by prokaryotic expression and purification. The DNA substrate, the protein and unlabeled single-stranded capture DNA were thoroughly mixed well in a buffer containing ATP and Mg²⁺, and incubated at 30°C for 60 minutes, and then a microplate reader was used to measure FAM fluorescence in real time. Mock was uses as a negative control, and a positive control was such that the same concentration of 5'-end FAM fluorophore-labeled single-stranded DNA was used as the substrate. A scatter plot is drawn by using reaction time (Time, min) as the abscissa and fluorescence intensity (ΔRFI, a.u.) as the ordinate.
Fig. 4 shows a schematic diagram of a linker used for constructing a sequencing library, a: sense strand (top strand); b: antisense strand (bottom strand).
Fig. 5 shows a schematic diagram of a sequencing library containing BCH105, a: sense strand (top strand); b: antisense strand (bottom strand); c: double-stranded target fragment; d: BCH105; e: chol-ssDNA.
Fig. 6 shows the current changes resulted by perforation translocation of the nucleic acid fragment to be sequenced.
Fig. 7 shows the results of comparative experiments for the helicase BCH105 and the helicase DDA known in the art under the same conditions. (A) shows the statistical results of the speed of DNA library passing through pores under the control of BCH105, (B) shows the statistical results of the speed of DNA library passing through pores under the control of DDA.

### Specific Models for Carrying Out the present invention

The technical solution in the examples of the present application will be clearly and completely described below with reference to the accompanying drawings of the present application. Those skilled in the art should understand that the examples are only for illustrative purposes and do not in any way limit the protection scope of the present application. Based on the examples of the present application, those skilled in the art can determine that equivalents of the examples are also within the protection scope of the present application.

Those skilled in the art should also understand that, unless otherwise stated, the cells or strains, plasmids, reagents, etc. used in the examples are all commercially available.

The inventors screened out two helicase BCH1X sequences from the deep sea metagenomic library (derived from the Shenzhen National Gene Bank): SEQ ID NO: 1, named BCH105; and SEQ ID NO: 2, named BCH178. Both the helicases contain the following amino acid fragment:
GTIHX FLNLKLDXGF Y1DDGY2ADNVY2 TKXKLVY3NKY4 NECL
wherein: X represents any amino acid residue; Y1=A or G; Y2=T or S; Y3=V or L; Y4=F or Y. The inventors discovered that the amino acid fragment is a key region that enables the helicase to perform better in sequencing.
Amino acid sequence of BCH105 (SEQ ID NO: 1): 
mkhdlssateeqryiftdfikqaqlalkgdsdyevfaiqgfagsgktwlsaliidellelgmkvavtspthkavrvslnmlknngi
dtnsplmypgtihhflnlkldhgfaddgtacnvttkaklvvnkfnealeyvdvlivdeasmvsgelydhalktlgdrakiilfigdsyqll
 
pvddedssiflkddifhykltkvvrqaedniiiaksqelikamdqktyypsvndyfvnitedtegikllksnvelfelyfsdfkdkmtga
ytnkvvnqfneyirytlyqetkfiadkdelvfqetytdskgniivsngeiievataklttdidkfkiwkivskknelgeavrfnvldpssyn
efndlldkycadakiakgydrskawkkyfklkekyakvrynfsstihklqgstyqnmyfdmrgldyfyrmnrdnvlrlvyvgitras
dqvfilqd
Amino acid sequence of BCH178 (SEQ ID NO: 2):

mvkdinkanekqqeafngiveaaknieekitkvyslvgsagtgktwtltqiikelldsgikvamttpthkalavvtdmlkeaki
ddpnlitgtihyflnlkldygfgddgsadnvstkpklvlnkyneclqytdlliidessmiseelykltmeilddrckmilfvgdkyqllpv
eggeniiydhpeimhyqltetvrqkagstiiekaneirdyikygnhpdnvfnlfhetdeiqmlqesdflpsyfanehnktigsftnamv
dqynnyvryvvtneldylanddevvfqkpysnasgdlifqngetvviestkkvfddknsvwywrckgkarmfnvldpdslsvykd
klkelldyaktqkgyhksnawkayfklmnrfgiikysfastlhklqgsttesmyfdmrdlkrfynrdkegvlrliyvaitrpseklfilgl

### Example 1: Cloning, expression and purification of BCH105

### Cloning and expression of BCH105

The full-length DNA sequence of BCH105 was ligated into PET.28a(+) plasmid, and the double enzyme cleavage sites as used were Nde1 and Xho1. As such, the N-terminal of the BCH105 protein as expressed had a 6*His tag and a thrombin enzyme cleavage site.

The constructed PET.28a(+)-BCH105 plasmid was transformed into *Escherichia coli* expression strain BL21(DE3) or derived bacteria thereof. A single colony was picked, added to 5 mL of LB medium containing kanamycin, and cultured overnight at 37°C under shaking. Then it was transferred to 1 L of LB medium (containing kanamycin), cultured under shaking at 37°C until OD600=0.6 to 0.8, then cooled to 16°C, and added with IPTG at a final concentration of 500 µM to induce expression overnight.

### Purification of BCH105

Buffer A: 20 mM Tris-HCl pH 7.5, 250 mM NaCl, 20 mM imidazole;
Buffer B: 20 mM Tris-HCl pH 7.5, 250 mM NaCl, 300 mM imidazole;
Buffer C: 20 mM Tris-HCl pH 7.5, 50 mM NaCl;
Buffer D: 20 mM Tris-HCl pH 7.5, 1000 mM NaCl;
Buffer E: 20 mM Tris-HCl pH 7.5, 100 mM NaCl;
*Escherichia coli* cells expressing BCH105 were collected, resuspended in Buffer A, and disrupted with a cell disrupter, and then centrifuged to obtain the supernatant. The supernatant was mixed with the Ni-NTA packing that had been previously equilibrated with Buffer A, and allowed binding for 1 hour. The packing was collected and washed extensively with Buffer A until no impurity proteins were washed out. Buffer B was then added to the packing to elute BCH105. The eluted BCH105 protein passed through a desalting column equilibrated with Buffer C to perform buffer replacement. Upon addition of an appropriate amount of thrombin, it was added to a ssDNA cellulose packing equilibrated with Buffer C, and digestion and binding were performed overnight at 4°C. The ssDNA cellulose packing was collected, washed 3 to 4 times with Buffer C, and then eluted with Buffer D. The protein purified by the ssDNA cellulose packing was concentrated and loaded onto a molecular sieve Superdex 200, in which the molecular sieve buffer as used was Buffer E. The target protein peak was collected, concentrated, and cryopreserved.

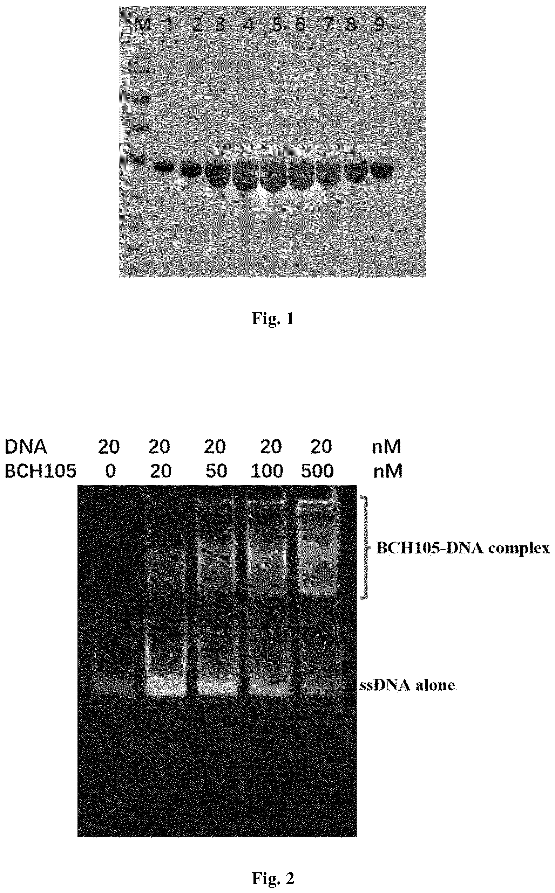

It could be seen from Fig. 1 that after purification, a larger amount of BCH105 protein with good purity could finally be obtained, in which an average of about 3 mg of the target protein was purified from per gram of bacteria. In comparison, it was much higher than the yield of the helicase DDA expressed in the same *Escherichia coli* under the same condition using the same expression vector (the helicase DDA was a commonly used helicase in this field, and only 0.23 mg of DDA protein was obtained from per gram of bacteria). Fractions 6 to 9 were selected and combined for subsequent experiments.
Amino acid sequence of the helicase DDA (SEQ ID NO: 3):

MTFDDLTEGQ KNAFNIVMKA IKEKKHHVTI NGPAGTGKTT LTKFIIEALI STGGTGIILA
APTHAAKKIL SKLSGKEAST IHSILKINPV TYEENVLFEQ KEVPDLAKCR VLICDEVSMY
DRKLFKILLS TIPPWCTIIG IGDNKQIRPV EPGENTAYIS PFFTHKDFYQ CELTEVKRSN
APIIDVATDV RNGKWNYDKV VDGHGVRGFT GDTALRDFMV NYFSIVKSLD DLFENRVMAF
TNKSVDKLNS IIRKKIFETD KDFIVGEIIV MQEPLFKTYK IDGKPVSEII FNNGQLVRII
EAEYTSTFVK ARGVPGEYLI RHWDLTVETY GDDEYYREKI KIISSDEELY KFNLFLAKTA
ETYKNWNKGG KAPWSDFWDA KSQFSKVKAL PASTFHKAQG MSVDRAFIYT PCIHYADVEL
AQQLLYVGVT RGRYDVFYV* (* indicated termination)

### Example 2: Detection of DNA binding and helicase activity of BCH105

### Detection of DNA binding ability of BCH105:

50 µL of reaction system: The ssDNA substrate and BCH105 protein were thoroughly mixed in a reaction buffer A. The final concentration of the DNA substrate (ssDNA) was 20 nM, and the final concentrations of the BCH105 protein used were 0 nM, 20 nM, 50 nM, 100 nM, and 500 nM, in which the reaction buffer A was: 50 mM HEPES, 100 mM KCl, pH 8.0.

The ssDNA substrate used was a 3' cy3-labeled single-stranded DNA, and its sequence was TTTTTTTTTTTTCTGAATCACGTACTATATGACACAGTAAAT-cy3.

The reaction was performed at room temperature for 1 h, and the sample was taken to perform 10% non-denaturing PAGE. The results were shown in Fig. 2.

The experimental results in Fig. 2 showed that as more BCH105 protein was added to the ssDNA sample, more and more cy3-labeled ssDNA bands migrated upward on the non-denaturing PAGE, indicating the formation of ssDNA-BCH105 complexes, thus proving BCH105 had a good ssDNA-binding ability.

### Detection of DNA unwinding ability of BCH105:

40 µL reaction system: The double-stranded DNA substrate with a 5'-overhang, BCH105 protein, and unlabeled single-stranded capture DNA were thoroughly mixed in reaction buffer B. The final concentration of the DNA substrate was 20 nM, the final concentration of the protein was 100 nM, and the final concentration of the captured DNA was 400 nM. The reaction buffer B was: 470 mM KCl, 25 mM HEPES, 2 mM ATP, 10 mM MgCl₂, pH 8.0.

The double-stranded DNA with a 5'-overhang was formed by annealing a single-stranded DNA labeled by BHQ-1 quenching group at the 3' end to a single-stranded DNA labeled by FAM fluorophore at the 5' end, which sequences were respectively:
5'-GCACCGAACTAGCAGCGTCGAAAAGCAGTACTTAGGCATT-BHQ-1-3',

5'-FAM-TTTTTTTTTTTTTTTTTTTTAATGCCTAAGTACTGCTTTTCGACGCTGCTAG
TTCGGTGC-3'.

The sequence of the unlabeled single-stranded capture DNA was:
5'-AATGCCTAAGTACTGCTTTTCGACGCTGCTAGTTCGGTGC-3'.

In the positive control group (positive), the single-stranded DNA labeled by FAM fluorophore at the 5' end was used instead of the double-stranded DNA with a 5'-overhang, and the rest were the same as in the experimental group.

In the negative control group (negative), nuclease-free water was used instead of the protein, and the rest were the same as in the experimental group.

40 µL of the reaction solution was added to the microplate, and the FAM fluorescence (excitation wavelength: 492 nm, emission wavelength: 518 nm) was measured in real time using a microplate reader. The temperature was 30°C, and the total time was 30 min. Each group of samples was repeated in 3 replicate wells.

The experimental results were shown in Fig. 3: the fluorescence value of the positive control group (positive) remained unchanged during the measurement process, approximately 9500; the fluorescence value of the negative control group (negative) remained unchanged during the measurement process, approximately 2600; the fluorescence value of the BCH105 experimental group gradually increased with the reaction time, that was, the fluorescence value of the BCH105 experimental group increased from 3200 (0 min) to 5600 (30 min). The experimental results in Fig. 3 showed that BCH105 had the activity of unwinding double-stranded DNA, and the unwinding direction was 5' to 3'.

### Example 3: Characterization and control of nucleic acids using BCH105

### Characterization and control of nucleic acids using BCH105

Two partially complementary DNA strands (sense strand (top strand) and antisense strand (bottom strand)) were annealed to form a linker (as shown in Fig. 4), which was ligated to the target double-stranded nucleic acid fragment to be sequenced (SEQ ID NO: 4) using T4 DNA ligase and purified at room temperature to prepare a sequencing library (as shown in Fig. 5). The sequencing library was then incubated with BCH105 for 1 hour at 25°C (at a molar concentration ratio of 1:8) to form a sequencing library containing BCH105 helicase, which was then incubated with a single-stranded DNA containing cholesterol at the 5' end (chol-ssDNA) for 10 minutes at room temperature. The sequence of the chol-ssDNA was complementary to a partial region of the antisense strand of the linker (bottom strand). When cholesterol bound to the phospholipid membrane, it could reduce the loading amount of the library and improve the capture rate.

In this experiment, a patch-clamp amplifier or other electrical signal amplifier was used to collect current signals.

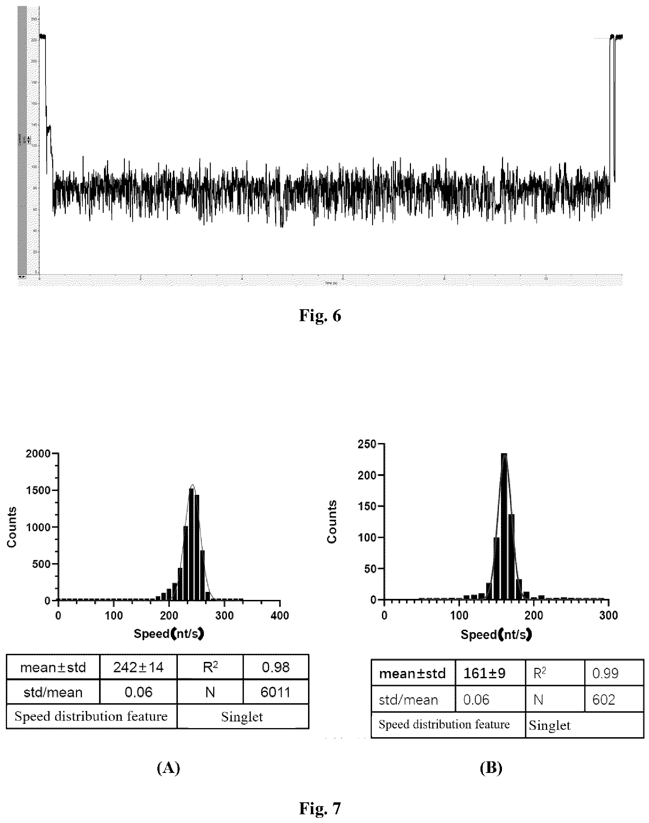

A Teflon membrane with micron-sized pores (50 to 200 µm in diameter) therein was used to divide the electrolytic cell into two chambers, i.e., cis chamber and trans chamber. A pair of Ag/AgCl electrodes was placed in each of the cis chamber and the trans chamber. After forming a bimolecular phospholipid membrane at the micropores of the two chambers, a nanometer porin (*Mycobacterium smegmatis* porin A (MspA, SEQ ID NO: 5), prepared according to Example 5) was added; after a single nanometer porin was inserted into the phospholipid membrane, electric measurement was performed. After applying 180 mV, the open pore current of a single channel was obtained. An appropriate amount of the sequencing library containing BCH105 helicase was added, and after waiting for a few minutes, the sequencing library was captured by the nanopores and the nucleic acids passed through the nanopores under the control of the helicase. In this experiment, 180 mV was applied to observe and obtain changes in the current signal. The buffer used in this experiment was: 0.47M KCl, 50 mM HEPES, 5 mM ATP, 25 mM MgCl₂, pH 7.6. The experimental results were shown in Fig. 6.

It could be seen from the results in Fig. 6 that the open pore current was about 220 pA. As the single strand of the DNA in the sequencing library entered into the nanopores, under the unwinding effect of BCH105, the DNA passed through the nanopores, partial current was blocked, and the current became smaller. Since different nucleotides had different sizes, the magnitudes of the blocked currents were also different, so that fluctuating current signals could be seen.

Those skilled in the art would understand that the unwinding activity and sequencing experimental results of BCH105 verified in this example could indirectly prove its stability. 0.9% NaCl in physiological saline was about 0.15M salt concentration, which was similar to salt concentration under a physiological condition, while the KCl concentration used in the DNA unwinding process in this example was 0.47M, which could prove that the helicase of the present application had high salt tolerance relative to the physiological environment (for example, it could tolerate 0.3 to 1M KCl).
Sequence of sense strand (Top strand) of Linker: 
5'-TTTTTTTTTTTTTTTTTTTTTTTTTTTTTTTTTTTTTTTTTTTTTTTTTTYYYYGGTTGTTTCTGTT
GGTGCTGATATTGCT-3' (Y = iSP18)
Sequence of antisense strand (Bottom strand) of Linker: 5'-GCAATATCAGCACCAACAGAAACAACCTTTGAGGCGAGCGGTCAA-3'
Sequence of chol-ssDNA: 5'-cholesterol-TTGACCGCTCGCCTC-3'
Sequence of target nucleic acid to be sequenced (SEQ ID NO: 4):

TCGCGCGTTTCGGTGATGACGGTGAAAACCTCTGACACATGCAGCTCCCGGAGA
CGGTCACAGCTTGTCTGTAAGCGGATGCCGGGAGCAGACAAGCCCGTCAGGGCGCG
TCAGCGGGTGTTGGCGGGTGTCGGGGCTGGCTTAACTATGCGGCATCAGAGCAGAT
TGTACTGAGAGTGCACCATATGCGGTGTGAAATACCGCACAGATGCGTAAGGAGAA
AATACCGCATCAGGCGCCATTCGCCATTCAGGCTGCGCAACTGTTGGGAAGGGCGA
TCGGTGCGGGCCTCTTCGCTATTACGCCAGCTGGCGAAAGGGGGATGTGCTGCAAG
GCGATTAAGTTGGGTAACGCCAGGGTTTTCCCAGTCACGACGTTGTAAAACGACGG
CCAGTGAATTCGAGCTCGGTACCTCGCGAATGCATCTAGATATCGGATCCCGGGCCC
GTCGACTGCAGAGGCCTGCATGCAAGCTTGGCGTAATCATGGTCATAGCTGTTTCCT
GTGTGAAATTGTTATCCGCTCACAATTCCACACAACATACGAGCCGGAAGCATAAA
GTGTAAAGCCTGGGGTGCCTAATGAGTGAGCTAACTCACATTAATTGCGTTGCGCTC
ACTGCCCGCTTTCCAGTCGGGAAACCTGTCGTGCCAGCTGCATTAATGAATCGGCCA
ACGCGCGGGGAGAGGCGGTTTGCGTATTGGGCGCTCTTCCGCTTCCTCGCTCACTGA

CTCGCTGCGCTCGGTCGTTCGGCTGCGGCGAGCGGTATCAGCTCACTCAAAGGCGGT
AATACGGTTATCCACAGAATCAGGGGATAACGCAGGAAAGAACATGTGAGCAAAA
GGCCAGCAAAAGGCCAGGAACCGTAAAAAGGCCGCGTTGCTGGCGTTTTTCCATAG
GCTCCGCCCCCCTGACGAGCATCACAAAAATCGACGCTCAAGTCAGAGGTGGCGAA
ACCCGACAGGACTATAAAGATACCAGGCGTTTCCCCCTGGAAGCTCCCTCGTGCGCT
CTCCTGTTCCGACCCTGCCGCTTACCGGATACCTGTCCGCCTTTCTCCCTTCGGGAAG
CGTGGCGCTTTCTCATAGCTCACGCTGTAGGTATCTCAGTTCGGTGTAGGTCGTTCGC
TCCAAGCTGGGCTGTGTGCACGAACCCCCCGTTCAGCCCGACCGCTGCGCCTTATCC
GGTAACTATCGTCTTGAGTCCAACCCGGTAAGACACGACTTATCGCCACTGGCAGCA
GCCACTGGTAACAGGATTAGCAGAGCGAGGTATGTAGGCGGTGCTACAGAGTTCTT
GAAGTGGTGGCCTAACTACGGCTACACTAGAAGAACAGTATTTGGTATCTGCGCTCT
GCTGAAGCCAGTTACCTTCGGAAAAAGAGTTGGTAGCTCTTGATCCGGCAAACAAA
CCACCGCTGGTAGCGGTGGTTTTTTTGTTTGCAAGCAGCAGATTACGCGCAGAAAAA
AAGGATCTCAAGAAGATCCTTTGATCTTTTCTACGGGGTCTGACGCTCAGTGGAACG
AAAACTCACGTTAAGGGATTTTGGTCATGAGATTATCAAAAAGGATCTTCACCTAGA
TCCTTTTAAATTAAAAATGAAGTTTTAAATCAATCTAAAGTATATATGAGTAAACTT
GGTCTGACAGTTACCAATGCTTAATCAGTGAGGCACCTATCTCAGCGATCTGTCTAT
TTCGTTCATCCATAGTTGCCTGACTCCCCGTCGTGTAGATAACTACGATACGGGAGG
GCTTACCATCTGGCCCCAGTGCTGCAATGATACCGCGAGACCCACGCTCACCGGCTC
CAGATTTATCAGCAATAAACCAGCCAGCCGGAAGGGCCGAGCGCAGAAGTGGTCCT
GCAACTTTATCCGCCTCCATCCAGTCTATTAATTGTTGCCGGGAAGCTAGAGTAAGT
AGTTCGCCAGTTAATAGTTTGCGCAACGTTGTTGCCATTGCTACAGGCATCGTGGTG
TCACGCTCGTCGTTTGGTATGGCTTCATTCAGCTCCGGTTCCCAACGATCAAGGCGA
GTTACATGATCCCCCATGTTGTGCAAAAAAGCGGTTAGCTCCTTCGGTCCTCCGATC
GTTGTCAGAAGTAAGTTGGCCGCAGTGTTATCACTCATGGTTATGGCAGCACTGCAT
AATTCTCTTACTGTCATGCCATCCGTAAGATGCTTTTCTGTGACTGGTGAGTACTCAA
CCAAGTCATTCTGAGAATAGTGTATGCGGCGACCGAGTTGCTCTTGCCCGGCGTCAA
TACGGGATAATACCGCGCCACATAGCAGAACTTTAAAAGTGCTCATCATTGGAAAA
CGTTCTTCGGGGCGAAAACTCTCAAGGATCTTACCGCTGTTGAGATCCAGTTCGATG
TAACCCACTCGTGCACCCAACTGATCTTCAGCATCTTTTACTTTCACCAGCGTTTCTG
GGTGAGCAAAAACAGGAAGGCAAAATGCCGCAAAAAAGGGAATAAGGGCGACACG
GAAATGTTGAATACTCATACTCTTCCTTTTTCAATATTATTGAAGCATTTATCAGGGT
TATTGTCTCATGAGCGGATACATATTTGAATGTATTTAGAAAAATAAACAAATAGGG
GTTCCGCGCACATTTCCCCGAAAAGTGCCACCTGACGTCTAAGAAACCATTATTATC
ATGACATTAACCTATAAAAATAGGCGTATCACGAGGCCCTTTCGTC

### Example 4: Comparison of the helicase BCH105 and the helicase DDA

Comparative experiments were conducted using the helicase BCH105 of the present application and the helicase DDA known in the art under the same conditions (the same as the experimental conditions of Example 3). The results were shown in Fig. 7, wherein, the speed of passing pore = length of target nucleic acid sequence / time of passing pore, and the time of passing pore was the length of time during which current signal change was observed. As shown in Fig. 7, the speed of passing pore was statistically calculated for the target DNA sequencing library. Fig. 7A showed the statistical results of the speed of passing pore for the DNA library under the control of BCH105, in which the average value was 242 nt/s; Fig. 7B showed the statistical results of the speed of passing pore for the DNA library under the control of DDA, in which the average value was 161 nt/s. This indicated that the speed of passing pore for the DNA library under the control of BCH105 was faster, suggesting that the helicase of the present application had higher sequencing speed, better salt tolerance and better stability.

### Example 5: Preparation of MspA protein

### Construction of expression vector for MspA protein

Through the In-fusion method, after digestion with NdeI and XhoI, the gene sequence encoding MspA protein was inserted into the cloning region of the vector pET24a. As such, the N-terminal of the expressed MspA protein had 6*His, which could be used as a purification tag, kanamycin was used for screening, and the constructed expression vector was named pET24a-MspA.

### Culture of recombinant Escherichia coli strain and induction of protein expression

LB liquid medium: tryptone 10g/L, yeast extract 5g/L, NaCl 10g/L.

The recombinant expression vector pET24a-MspA was transformed into the expression strain *E. coli* BL21 (DE3). The bacterial solution was spread evenly on an LB solid culture plate with 50 µg/mL kanamycin, and cultured at 37°C overnight. Single colony was picked and cultured in 5 ml of LB medium (containing 50 µg/mL kanamycin) at 37°C and 200 rpm overnight. The bacterial solution obtained was inoculated into 50 ml of LB (containing 50 µg/mL kanamycin) at a ratio of 1:100 and cultured at 37°C and 200 rpm for 4 hours. The expanded cultured bacterial solution was inoculated into 2L of LB (containing 50 µg/mL kanamycin) at a ratio of 1:100 and cultured at 37°C and 200 rpm. When the OD600 value reached about 0.6 to 0.8, IPTG was added at a final concentration of 0.5mM, and the bacteria were cultured at 18°C and 200rpm overnight (about 16 to 18 hours). The grown bacterial cells were collected by centrifugation at 8000 rpm, and the bacterial cells were frozen at -20°C for later use.

### Extraction and purification of recombinant MspA protein

### Preparation of purification buffer

### 1. Ni column affinity chromatography

Buffer A1 (equilibration buffer): 20mM Tris-HCl + 250mM NaCl + 0.5% Tween-20 + 5% glycerol, pH 7.9;
Buffer B1 (elution buffer): 20mM Tris-HCl + 250mM NaCl + 0.5% Tween-20 + 5% glycerol + 500mM imidazole, pH 7.9.

### 2. Ion exchange chromatography

### Dilution buffer:

Buffer C1 (equilibration buffer): 20mM Tris-HCl + 50mM NaCl + 0.5% Tween-20 + 5% glycerol, pH 6.5.
Buffer D1 (elution buffer): 20mM Tris-HCl + 1000mM NaCl + 0.5% Tween-20 + 5% glycerol, pH 6.5.

### 3. Diluent of protein sample

Buffer E1 (diluent): 20mM Tris-HCl + 0.5% Tween-20 + 5% glycerol, pH 6.5.

The recombinant *Escherichia coli* cells were resuspended at a ratio of 1 g of recombinant *Escherichia coli* cells to 10 ml of Buffer A1, and the cells were disrupted by ultrasonic until the cell solution became clear. The disrupted cells were centrifuged at 12,000 rpm and 4°C for 30 min. The supernatant was taken, filtered with a 0.22 µm filter membrane, and stored at 4°C.

The column of Ni column affinity chromatography was washed with water of 5 column volumes (5CV), washed with buffer B1 of 5CV, and equilibrated with buffer A1 of 10CV before sample loading. After the sample loading was completed, equilibration was performed with buffer A1 of 15CV, then buffer B1 was used for linear elution (0 to 8% buffer B1, 30CV) to remove impurities, and buffer B1 was used for linear elution (8 to 100% buffer B1 , 5CV) to collect the target protein.

The protein collected by the Ni column was diluted 2 times with buffer E1. The anion exchange Q column was washed with water for 5CV, and equilibrated with buffer C1 for 5CV, and then the protein sample was loaded. After equilibration with buffer C1 for 5CV, linear elution was performed with elution buffer D1 (0% to 9% buffer D1, 30CV), and then linear elution was performed with elution buffer D1 (9% to 100% buffer D1, 10CV), so as to collect the protein. The collected protein was dialyzed overnight at 4°C with buffer A1 as dialysis solution, and then stored at -80°C.
Amino acid sequence of MspA protein (SEQ ID NO: 5):

GLDNELSLVDGQDRTLTVQQWDTFLNGVFPLDRNRLTREWFHSGRAKYIVAGPGA
DEFEGTLELGYQIGFPWSLGVGINFSYTTPNILIDDGDITRPPFGLNSVITPNLFPGVSISAD
LGNGPGIQEVATFSVDVSGAEGGVAVSNAHGTVTGAAGGVLLRPFARLIASTGDSVTTY
GEPWNMN

The preferred embodiments of the present application have been described in detail above. However, the present application is not limited to the specific details of the above embodiments. Within the scope of the technical concept of the present invention, those skilled in the art can make various changes to the technical solutions of the present application and the required technical effects can still be obtained, and these changes all belong to the protection scope of the present application.

## Claims

1. A helicase, which comprises:
(i) the amino acid sequence as set forth in SEQ ID NO: 1 or 2; or
(ii) an amino acid sequence that has a sequence identity of at least 80%, at least 85%, at least 90%, at least 91%, at least 92%, at least 93%, at least 94%, at least 95%, at least 96%, at least 97%, at least 98%, or at least 99% as compared to the amino acid sequence as set forth in SEQ ID NO: 1 or 2 and has a helicase activity; or
(iii) an amino acid sequence that has no more than 20, 15, 10, 5, 4, 3, 2 or 1 amino acid difference as compared to the amino acid sequence as set forth in SEQ ID NO: 1 or 2 and has a helicase activity.

2. The helicase according to claim 1, which comprises the following amino acid fragment:
GTIHX FLNLKLDXGF Y1DDGY2ADNVY2 TKXKLVY3NKY4 NECL
wherein: X represents any amino acid residue; Y1 represents A or G; Y2 represents T or S; Y3 represents V or L; Y4 represents F or Y.

3. The helicase according to claim 1, wherein the amino acid difference comprises an amino acid substitution, deletion and/or insertion or N-terminal and/or C-terminal extension, preferably, the amino acid substitution is a conservative amino acid substitution.

4. The helicase according to claim 1, which consists of the amino acid sequence as set forth in SEQ ID NO: 1 or 2.

5. A nucleotide sequence encoding the helicase according to any one of claims 1 to 4.

6. A recombinant vector comprising the nucleotide sequence according to claim 5, which preferably is a recombinant expression vector.

7. A cell comprising the nucleotide sequence according to claim 5 or the recombinant vector according to claim 6, wherein preferably, the cell is a prokaryotic cell or a eukaryotic cell; more preferably, the cell is an *Escherichia coli* cell, a yeast cell, an insect cell or a mammalian cell.

8. A complex structure, which comprises the helicase BCH1X according to any one of claims 1 to 4 and a binding moiety for binding to a polynucleotide.

9. The complex structure according to claim 8, wherein the binding moiety is a binding moiety capable of binding to a base of a polynucleotide, and/or a binding moiety capable of binding to a sugar of a polynucleotide, and/or a binding moiety capable of binding to a phosphate in a polynucleotide.

10. A method for controlling and characterizing a target polynucleotide, wherein the method comprises the following steps:
(a) contacting a target polynucleotide with a pore, and the helicase according to any one of claims 1 to 4 or the complex structure according to claim 8, such that the helicase or complex structure can control movement of the target polynucleotide through the pore; and
(b) obtaining one or more characteristics of a nucleotide in the target polynucleotide when it interacts with the pore, thereby characterizing the target polynucleotide;
wherein, the one or more characteristics is selected from the group consisting of a change in current signal magnitude, a change in current signal duration, a change in voltage signal magnitude, and a change in voltage signal duration.

11. The method according to claim 10, wherein the method is a single-molecule nanopore sequencing method.

12. A kit for controlling and characterizing a polynucleotide, wherein the kit comprises the helicase according to any one of claims 1 to 4 or a complex structure thereof, and a pore.

13. A kit for a single-molecule nanopore sequencing, wherein the kit comprises the helicase according to any one of claims 1 to 4 or a complex structure thereof, and a pore.

14. A sensor for characterizing a target polynucleotide, wherein the sensor comprises a complex formed by a pore and the helicase according to any one of claims 1 to 4 or a complex structure thereof.

15. A device for characterizing a target polynucleotide, wherein the device comprises the helicase according to any one of claims 1 to 4 or a complex structure thereof, and a pore.

16. The method according to claim 10 or 11, the kit according to claim 12 or 13, the sensor according to claim 14 or the device according to claim 15, wherein the pore is a transmembrane pore, preferably a biological pore, a solid-state pore or a biological-solid-state hybrid pore; more preferably, the biological pore is selected from the group consisting of α-hemolysin protein (α-HL), *Mycobacterium smegmatis* porin A (MspA), curli-specific transport channel protein (CsgG), or type III secretion system protein (InvG).
